# EUROPEAN PATENT SPECIFICATION

(11) **EP 3 506 271 B1**
(45) Date of publication and mention of the grant of the patent: **08.05.2024**
(21) Application number: 17382917.7
(22) Date of filing: 28.12.2017
(51) Int. Cl.: G16H 10/65, G16H 10/60

(54) **COMMUNICATION SYSTEM MANAGING AN ELECTRONIC HEALTH RECORD**
KOMMUNIKATIONSSYSTEM ZUR VERWALTUNG EINES ELEKTRONISCHEN GESUNDHEITSDATENSATZES
SYSTÈME DE COMMUNICATION PERMETTANT DE GÉRER LE DOSSIER MÉDICAL ÉLECTRONIQUE

(43) Date of publication of application: 03.07.2019
(73) Proprietor: BULL SAS, 78340 Les Clayes-sous-Bois (FR)
(72) Inventor: CAVERO BARCA, Carlos, 28822 Coslada, Madrid (ES); RODRIGUEZ PEREZ, Juan Mario, 34005 Palencia (ES); QUINTERO PADRON, Ana Maria, 38320 La Higuerita, La Laguna (ES); RAMOS MAIA-MARTINS, Ivo, 2770-117 Paço d'Arcos, Oeiras (PT); ASO LETE, Santiago, 28039 Madrid (ES); JORDAN RODRIGUEZ, Blanca, 28034 Madrid (ES); PEREZ PEREZ, Manuel Marcelino, 28039 Madrid (ES)
(74) Representative: Plasseraud IP

(56) References cited:
- WO-A1-2017/180808
- US-A1- 2005 060 198
- US-A1- 2012 232 929
- US-A1- 2015 278 454

## Description

### FIELD OF THE INVENTION

The invention relates to the field of communication systems managing electronic health records.

### BACKGROUND OF THE INVENTION

According to a prior art, it is known to manage an electronic health record within a database owned and managed by a hospital. When a patient suffers from a new disease or is treated for this new disease or has cured from this new disease, the hospital where the patient has been treated updates the patient's electronic health record.

However, a first inconvenient of this prior art is that the patient may be treated for many diseases at different locations, by different doctors, in different hospitals, and that gathering all the medical data related to a specific patient within a single electronic health record is not that simple. A second inconvenient is that most of, or even all, different hospitals, and moreover most of, or even all, different medical actors, have different formats from one another to store medical data related to patients, and that storing all the medical data related to a specific patient within a single electronic health record in a coherent and consistent way is not straightforward either.

A huge and centralized and secured medical database, to which all medical actors, including hospitals and patients could connect, would be a possible solution. However, this huge and centralized database, especially if really secured, would be rather complex, to implement as well as to maintain, and rather expensive too.

US2015/278454A1 discloses a method and apparatus for managing a client's medical records in a mobile terminal of the client, in which a central server such as a cloud server does not exist in order to manage patients' medical records.

### SUMMARY OF THE INVENTION

The object of the present invention is to alleviate at least partly the above mentioned drawbacks.

More particularly, the invention aims to propose a way to get a secured, updated, and standardized, electronic health record.

Therefore, according to the invention, the management of electronic health record is shifted from medical information center to mobile terminal application dedicated to patient.

This shift would not be straightforward to the man skilled in the art for at least two technical reasons. The first technical reason is a capacity reason; indeed, how would it be possible to implement complex and sophisticated technical functions within a mobile terminal application without making it unreasonably complex and expensive, capacities of a mobile terminal being rather limited, even if daily improving? The second technical reason is a security reason; indeed, how would it be possible to store such sensitive medical data without being subject to patient's cheating and/or third party's piracy?

This shift is performed while keeping bearable the added complexity, to mobile terminal application, related to standardization and security of electronic health record update.

This shift is also performed while making easier the use, for the patient, of this updated electronic health record.

This shift is also performed while keeping a sufficient level of security, in particular with respect to possible patient's cheating.

This shift is performed by separating technical data and medical data from each other. Indeed, the hospitals and other medical actors keep the technical data with the associated technical complexity at their level, whereas only the medical data without this technical complexity are shifted at the mobile terminal level, that is to say at the patient user level.

This object is achieved with a communication system as defined in independent claim 1.

According to embodiments of the invention, this communication system is a patient-centered solution which enables patient empowerment in the management of its own medical information, storing their Electronic Health Record (EHR) and health data coming from different Hospital Information Systems (HIS).

According to embodiments of the invention, this communication system solves the need of current healthcare organizations into the adoption of healthcare data openness that follows a patient centered design and takes advantage of its potential benefits. Instead of having standardized repositories at the hospital facilities, the patient becomes the driver of the change bringing standardized pieces of its Electronic Health Record in his own mobile terminal 1, leading to a "distributed interoperability" within this communication system.

According to embodiments of the invention, this communication system gets potential benefits, from the analysis of the information generated by the patient throughout its complete care process between different healthcare stakeholders, which can be one or more among the following ones: an acceleration of clinical research, and/or more accessible and better developed measures of clinical performance, and/or increased public health monitoring and disease management, and/or new care delivery models, and/or patient self-management, and/or better care coordination across settings, and/or new data that supports decision making, and/or compliance of clinical or medical information with the "distributed interoperability" within the communication system because the driver for the exchange of medical data is the patient himself.

Preferred embodiments comprise one or more of the following features, which can be taken separately or together, either in partial combination or in full combination, with any of previously cited objects of the invention.

Preferably, said medical information centers are managed by one or more hospitals.

Hence, since hospitals often have proprietary data formats making data sharing and data aggregation rather hard and burdensome to implement, this central management at mobile terminal application level will be all the more efficient and interesting.

Preferably, said electronic health record is not stored in full in either of said medical information centers, preferably is not stored in full in any other medical information center managed by a hospital.

Hence, this unique patient oriented electronic health record is simpler to manage for the mobile terminal application and simpler to use for the patient.

According to the claimed invention, said first format converter also converts from said standard format into said first specific format, said second format converter also converts from said standard format into said second specific format, and wherein it is implemented so that: said first medical information center can download, in an ordinary mode, from updated electronic health record of said mobile terminal application, in standard format, a shared part of said second medical data via a third request sent through said first gateway, and can store, in first specific format, said shared part of second medical data, in said first medical information center, so as to update said first medical information center, said first medical information center cannot download, in an ordinary mode, from updated electronic health record of said mobile terminal application, in any format, a secret part of said second medical data, said second medical information center can download, in an ordinary mode, from updated electronic health record of said mobile terminal application, in standard format, a shared part of said first medical data via a fourth request sent through said second gateway, and can store, in second specific format, said shared part of first medical data, in said second medical information center, so as to update said second medical information center, said second medical information center cannot download, in an ordinary mode, from updated electronic health record of said mobile terminal application, in any format, a secret part of said first medical data, said patient can consult both shared and secret parts of both first and second medical data in said updated electronic health record via said mobile terminal application.

Hence, privacy of data, and especially privacy of very sensitive data, is respected, even regarding hospitals' consulting them, medical data being disclosed only on a "need to know" basis, even to hospitals.

According to the claimed invention, the communication system is implemented so that said first medical information center can download, in an urgency mode, from updated electronic health record of said mobile terminal application, in standard format, both said shared and secret parts of said second medical data via a fifth request sent through said first gateway, so as to deal with an urgency with said patient, said second medical information center can download, in an urgency mode, from updated electronic health record of said mobile terminal application, in standard format, both said shared and secret parts of said first medical data via a sixth request sent through said second gateway, so as to deal with an urgency with said patient.

Hence, a good compromise is proposed between respecting medical data privacy most of the time, even regarding medical data communication towards hospitals, and disclosing needed confidential medical data in case of urgency, again regarding medical data communication towards hospitals.

Preferably, communication system is implemented so that said mobile terminal application manages electronic health records respectively dedicated to different patients, in standard format, each patient can only consult his own updated electronic health record via said mobile terminal application but cannot consult any part of any medical data of another's patient updated electronic health record via said mobile terminal application.

Hence, privacy of data, and especially privacy of very sensitive data, is fully respected, in particular regarding other patients.

Preferably, each of either medical information centers comprises: a storage device storing said medical data, in said specific format, a connector including said format converter, adapted to get said medical data from said storage device, in said specific format, and to convert them into said standard format, before transmitting them, a desktop adapted, to request from said connector converted patient's medical data transmission, and to further transmit them to said patient's mobile terminal preferably wireless.

Hence, most of intelligence required to transform and to transmit medical data is located at medical information center premises, thereby making much simpler the mobile terminal application running on patient's mobile terminal.

Preferably, said connector includes said format converter which is adapted to receive said medical data from said desktop, in said standard format, and to convert them into said specific format, before transmitting them to said storage device, said desktop is adapted, to transmit to said connector patient's medical data, after having received them from said patient's mobile terminal preferably wireless.

Hence, most of intelligence required to transform and to transmit medical data is located at medical information center premises, thereby making much simpler the mobile terminal application running on patient's mobile terminal.

Preferably, said wireless communication is Bluetooth or Near Field Communication.

Hence, Bluetooth (registered trademark) or Near Field Communication are efficient wireless communication protocols supported by most of existing mobile terminals.

Preferably, said wireless communication is direct without going through any public network, preferably without going through any network.

Hence, this drastically reduces the risk of medical data piracy, so notably increases security, because of the geographical proximity between medical information center and mobile terminal during medical data transfer.

Preferably, at least said first and second requests, preferably at least first and second and third and fourth requests, more preferably all requests, are performed by filling and sending of predetermined templates.

This way, not only is the communication made simpler, but also and above all, the sensitive medical data transmission can be more easily controlled, to the cost of more constrained query syntax.

Preferably, at least one of said specific format is SOAP or REST or FILE, preferably said specific formats are chosen in a list consisting of "SOAP, REST, FILE".

Preferably, said standard format is XML or JSON.

Preferably, medical information center comprises: a storage device storing said medical data, in said specific format, a connector including said format converter, adapted to get said medical data from said storage device, in said specific format, and to convert them into said standard format, before transmitting them, a desktop adapted, to request from said connector converted patient's medical data transmission, and to further transmit them to said patient's mobile terminal preferably wireless.

Hence, most of intelligence required to transform and to transmit medical data is located at medical information center premises, thereby making much simpler the mobile terminal application running on patient's mobile terminal.

Preferably, said medical information center is managed by a hospital, said electronic health record is not stored in full in said medical information center.

Hence, since hospitals often have proprietary data formats making data sharing and data aggregation rather hard and burdensome to implement, this unique patient oriented electronic health record is simpler to manage and simpler to use for the patient.

Further features and advantages of the invention will appear from the following description of embodiments of the invention, given as non-limiting examples, with reference to the accompanying drawings listed hereunder.

### BRIEF DESCRIPTION OF THE DRAWINGS

Fig. 1 shows an example of a global and high level sketch of the communication system according to an embodiment of the invention.
Fig. 2 shows an example of a global architecture of the communication system according to an embodiment of the invention.
Fig. 3 shows an example of an implementation of a data retrieving operation from a hospital information system within the communication system according to an embodiment of the invention.
Fig. 4 shows an example of an implementation of a template configuration operation within the communication system according to an embodiment of the invention.

### DETAILED DESCRIPTION OF THE INVENTION

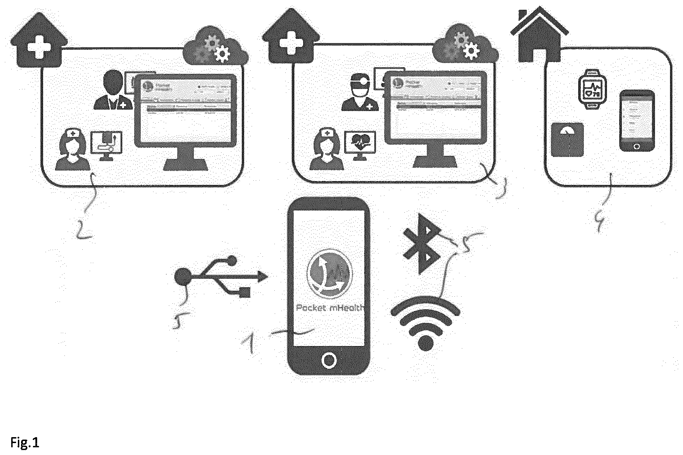

Fig. 1 shows an example of a global and high level sketch of the communication system according to an embodiment of the invention.

There is a mobile terminal 1, for example a mobile phone 1 or a mobile smart phone 1, which contains an electronic health record (EHR) dedicated to the patient to whom the mobile terminal 1 belongs. This mobile terminal 1, including storage of the electronic health record of the patient owner of this mobile terminal 1, may communicate, through several communication channels 5, including for example Bluetooth (registered trademark) or Near Field Communication, with several medical actors like a hospital information system 2 or like another hospital information system 3 or like a personal information system 4 like a home information system and/or other connected devices like tablets, watches or electronic medical devices.

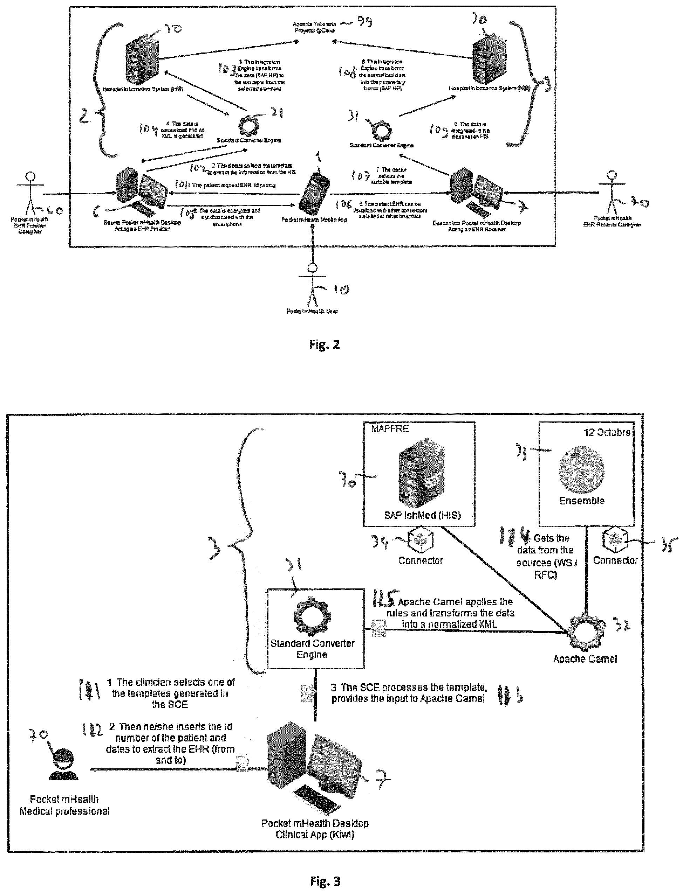

Fig. 2 shows an example of a global architecture of the communication system according to an embodiment of the invention.

The architecture of the communication system comprises, a mobile terminal 1 dedicated to a patient 10 and to his electronic health record, a source desktop 6 acting as an electronic health record provider and managed by an electronic health record provider caregiver 60 which is usually a doctor, a destination desktop 7 acting as an electronic health record receiver and managed by an electronic health record receiver caregiver 70 which is usually a doctor, an information hospital system 2 including a medical database 20 and a standard converter engine 21, an information hospital system 3 including a medical database 30 and a standard converter engine 31, a standard database 99 including information related to templates and standardized format medical data.

In step 101, at patient 10 request, mobile terminal 1 requests an update of its electronic health record to source desktop 6 managed by electronic health record provider caregiver 60, and authenticates by source desktop 6.

In step 102, the source desktop 6 managed by electronic health record provider caregiver 60 selects the template corresponding to mobile terminal 1 request in order to request corresponding medical data extraction from the hospital information system 2 by sending corresponding request to standard converter engine 21.

In step 103, standard converter engine 21 converts and transfers this request to medical database 20 linked to standard database 99. Medical database 20, with the help of standard database 99, fills in the requested template with proprietary medical data, transfers the filled template to standard engine converter 21.

In step 104, standard engine converter 21 normalizes and standardizes proprietary medical data so as to generate a standardized completed template, for example in XML format, and so as to transfer this standardized completed template to source desktop 6.

In step 105, this standardized completed template, filled in with standardized medical data, is encrypted by source desktop 6 and forwarded by source desktop 6 toward mobile terminal 1 so as to be synchronized with mobile terminal 1. The result of this step 105 is an updated electronic health record loaded in mobile terminal 1 at patient's request.

In step 106, at patient 10 request, mobile terminal 1 allows his electronic health record to be visualized, either directly or via other connectors installed at hospital information system 3, by destination desktop 7 managed by electronic health record receiver caregiver 70, and which destination desktop 7 extracts from this electronic health record suitable medical data to update hospital information system 3.

In step 107, the destination desktop 7 managed by electronic health record receiver caregiver 70 selects the suitable template updated with the medical data extracted from this electronic health record to forward it to standard converter engine 31.

In step 109, standard converter engine 31 converts and transfers this suitable template to medical database 30 linked to standard database 99.

In step 108, medical database 30, with the help of standard database 99, extracts the relevant medical data to update its own files in proprietary format with these new relevant medical data extracted from electronic health record stored on mobile terminal 1.

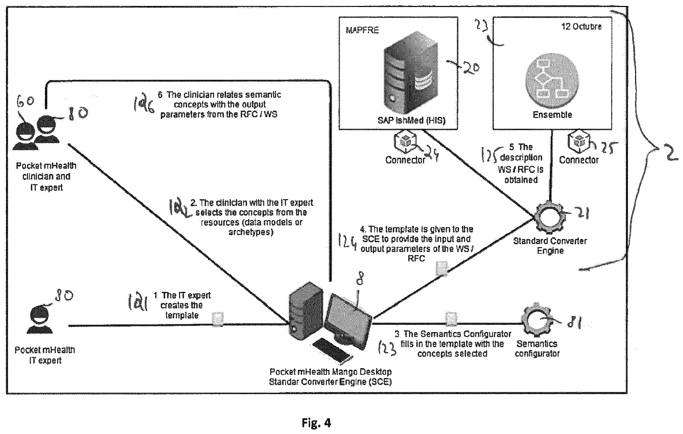

The communication system according to embodiments of the invention preferably comprises a patient oriented smartphone 1 application and a set of desktop 6 or 7 applications installed at healthcare stakeholders systems with access to hospital information systems 2 or 3 using associated connectors represented on figures 3 and 4. The mobile application allows carrying, accessing and transferring the personal medical information in the form of a standardized Electronic Health Record, therefore making use for example of different healthcare standards like "openEHR/EN113606" and "HL7 FHIR", and terminologies such as "SNOMED-CT". The tools installed at the hospital information systems 2 and 3 allow accessing them using configured templates, normalizing the clinical or medical data and transferring the electronic health record to the mobile smartphone 1 using wireless communication, for example Bluetooth (registered trademark). The mobile terminal 1 application can bring together the medical information coming from the different hospital information systems 2 and 3, and this is totally transparent to patients.

The communication system according to embodiments of the invention not only provides preferably the benefits of a connected practice or organization such as clinical or medical efficacy improvement, healthcare cost savings and increase of overall care quality, but also supports the care transformation that is enabled by a patient centered design.

The communication system according to embodiments of the invention will help the mobile smartphone 1 to become part of patient's body in a daily fashion in order to also modify the way the users take care of their health, giving the possibility to the citizens to always bring with them their Electronic Health Record in their mobile smartphone 1, offering meaningful advantages to the healthcare systems, such as interoperability, clinical efficiency improvement, medical errors reduction and healthcare costs savings.

Fig. 3 shows an example of an implementation of a data retrieving operation from a hospital information system within the communication system according to an embodiment of the invention.

Hospital information system 3 includes between standard engine converter 31 and medical database 30, an Apache Camel 32 and a connector 34 associated to medical database 30. Hospital information system 3 also includes a connector 35 associated to an additional medical database 33. Apache camel 32 and connectors 34 and 35 may alternatively be considered as part of standard engine converter 31.

In step 111, at the destination desktop 7 managed by electronic health record receiver caregiver 70 which can be a clinician, this clinician 70 selects one of the templates which can be generated by standard converter engine 31.

In step 112, clinician 70 first inserts the identification number of the patient to whom the contemplated electronic health record belongs, then inserts the date, and afterwards asks for extraction of medical data related to the contemplated electronic health record.

In step 113, standard converter engine 31 processes the selected template and forwards it as an input to Apache Camel 32.

In step 114, Apache Camel 32 gets the wished medical data from the medical database 30 and/or from the additional medical database 33, respectively via the connectors 34 and 35.

In step 115, Apache Camel 32 transforms the retrieved medical data into a standardized and normalized format, for example in XML format, by applying preexisting rules. Once received by standard engine converter 31, the standardized template filled with medical data retrieved from hospital information system 3 and related to contemplated electronic health record, is transmitted to the destination desktop 7 where the clinician 70 can visualize and consult them, and can thereafter use this standardized template filled with medical data retrieved from hospital information system 3 and related to contemplated electronic health record, in order to update this contemplated electronic health record stored in mobile terminal 1 not represented on figure 3.

The communication system according to embodiments of the invention preferably uses the dual model approach much separating static from dynamic information, or alternatively said, technical from clinical or medical data. This means that the technical people is in charge of the backend architecture allowing data models, like archetypes in "openEHR / EN13606" or like resources in "HL7 FHIR", to be processed on runtime. The figure 3 shows how the Graphical User Interfaces (GUI) are configured and the medical data are to be retrieved using predetermined templates.

Fig. 4 shows an example of an implementation of a template configuration operation within the communication system according to an embodiment of the invention.

Hospital information system 2 includes between standard engine converter 21 and medical database 20, a connector 24 associated to medical database 20. Hospital information system 2 also includes a connector 25 associated to an additional medical database 23. Connectors 24 and 25 may alternatively be considered as part of standard engine converter 21. There is also a creation desktop 8 to which is associated a semantics configurator 81.

In step 121, at the creation desktop 8 managed by both electronic health record caregiver 60, which can be a clinician, and technical expert 80, this technical expert 80 creates each one of the templates which will then be generated by standard converter engine 21 upon future request.

In step 122, both clinician 60 and technical expert 80 select the concepts from the existing resources which are for instance data models and archetypes, on creation desktop 8.

In step 123, semantics configurator 81 fills in the created template with the selected concepts.

In step 124, creation desktop 8 forwards the created and filled in template to standard converter engine 21, so that standard converter engine 21 can provide the needed input and output parameters to be exchanged with hospital information system 2.

In step 125, standard converter engine 21 gets the wished description of medical data from the medical database 20 and/or from the additional medical database 23, respectively via the connectors 24 and 25. Then, wished parameters are extracted therefrom and are transmitted by standard converter engine 21 to creation desktop 8.

In step 126, clinician 60 relates the semantic concepts with the output parameters got from the medical database 20 and/or from the additional medical database 23.

After all these steps are terminated, maybe with some iterations between the different actors of the communication system, the new template just created is then available for further use, both in standard format at one side of standard converter engine 21 and in proprietary format at the other side of standard converter engine 21.

Once the templates configured by the technical expert and the clinical or medical modeler, the guide user interface of the connector (Kiwi) can access and process the information coming from the hospital information system 2. The standard converter engine 21 will provide the SAP and WSDL connector.

The invention has been described with reference to preferred embodiments. However, many variations are possible within the scope of the claims.

## Claims

1. Communication system comprising:
➢ a mobile terminal (1) application managing an electronic health record dedicated to a patient (10), in a standard format,
➢ a first medical information center (2), storing first medical data related to said patient (10), in a first specific format,
➢ a second medical information center (3), storing second medical data related to said patient (10), in a second specific format,
wherein it also comprises:
➢ a first gateway (21), between said first medical information center (2) and said mobile terminal (1) application, including a first format converter from said first specific format into said standard format,
➢ a second gateway (31), between said second medical information center (3) and said mobile terminal (1) application, including a second format converter from said second specific format into said standard format,
wherein it is implemented so that:
➢ said mobile terminal (1) application can download, in standard format, said first medical data from said first medical information center (2) via a first request sent through said first gateway (21) and said second medical data from said second medical information center (3) via a second request sent through said second gateway (31), and can store, said first medical data and second medical data, in said electronic health record, so as to update said electronic health record,
➢ said patient (10) can consult said updated electronic health record via said mobile terminal (1) application,
➢ said patient (10) cannot, on its own, via said mobile terminal (1) application, modify or cancel, said first and second medical data, once stored in said mobile terminal (1) application, so as to make secure said updated electronic health record, the system being **characterized in that**:
➢ said first format converter also converts from said standard format into said first specific format,
➢ said second format converter also converts from said standard format into said second specific format,
and wherein it is implemented so that:
➢ said first medical information center (2) can download, in an ordinary mode, from updated electronic health record of said mobile terminal (1) application, in standard format, a shared part of said second medical data via a third request sent through said first gateway (21), and can store, in first specific format, said shared part of second medical data, in said first medical information center (2), so as to update said first medical information center (2),
➢ said first medical information center (2) cannot download, in an ordinary mode, from updated electronic health record of said mobile terminal (1) application, in any format, a secret part of said second medical data,
➢ said second medical information center (3) can download, in an ordinary mode, from updated electronic health record of said mobile terminal (1) application, in standard format, a shared part of said first medical data via a fourth request sent through said second gateway (31), and can store, in second specific format, said shared part of first medical data, in said second medical information center (3), so as to update said second medical information center (3),
➢ said second medical information center (3) cannot download, in an ordinary mode, from updated electronic health record of said mobile terminal (1) application, in any format, a secret part of said first medical data,
➢ said patient (10) can consult both shared and secret parts of both first and second medical data in said updated electronic health record via said mobile terminal (1) application,
and wherein it is implemented so that:
➢ said first medical information center (2) can download, in an urgency mode, from updated electronic health record of said mobile terminal (1) application, in standard format, both said shared and secret parts of said second medical data via a fifth request sent through said first gateway (21), so as to deal with an urgency with said patient (10),
➢ said second medical information center (3) can download, in an urgency mode, from updated electronic health record of said mobile terminal (1) application, in standard format, both said shared and secret parts of said first medical data via a sixth request sent through said second gateway (31), so as to deal with an urgency with said patient (10).

2. Communication system according to claim 1, wherein:
➢ said medical information centers (2, 3) are managed by one or more hospitals.

3. Communication system according to claim 2, wherein:
➢ said electronic health record is not stored in full in either of said medical information centers (2, 3), preferably is not stored in full in any other medical information center managed by a hospital.

4. Communication system according to any of preceding claims, wherein it is implemented so that:
➢ said mobile terminal (1) application manages electronic health records respectively dedicated to different patients, in standard format,
➢ each patient (10) can only consult his own updated electronic health record via said mobile terminal (1) application but cannot consult any part of any medical data of another's patient updated electronic health record via said mobile terminal (1) application.

5. Communication system according to any of preceding claims, wherein each of either medical information centers (2, 3) comprises:
➢ a storage device (20, 23, 30, 33) storing said medical data, in said specific format,
➢ a connector (24, 25, 34, 35) including said format converter, adapted to get said medical data from said storage device (20, 23, 30, 33), in said specific format, and to convert them into said standard format, before transmitting them,
➢ a desktop (6, 7) adapted, to request from said connector (24, 25, 34, 35) converted patient's medical data transmission, and to further transmit them to said patient's mobile terminal (1) preferably wireless.

6. Communication system according to claim 5, wherein:
➢ said connector (24, 25, 34, 35) includes said format converter which is adapted to receive said medical data from said desktop (6, 7), in said standard format, and to convert them into said specific format, before transmitting them to said storage device (20, 30),
➢ said desktop (6, 7) is adapted, to transmit to said connector (24, 25, 34, 35) patient's medical data, after having received them from said patient's mobile terminal (1) preferably wireless.

7. Communication system according to claim 5 or 6, wherein:
➢ said wireless communication is Bluetooth or Near Field Communication.

8. Communication system according to any of claims 5 to 7, wherein:
➢ said wireless communication is direct without going through any public network, preferably without going through any network.

9. Communication system according to any of preceding claims, wherein:
➢ at least said first and second requests, preferably at least first and second and third and fourth requests, more preferably all requests, are performed by filling and sending of predetermined templates.

10. Communication system according to any of preceding claims, wherein:
➢ at least one of said specific format is SOAP or REST or FILE,
➢ preferably said specific formats are chosen in a list consisting of "SOAP, REST, FILE".

11. Communication system according to any of preceding claims, wherein:
➢ said standard format is XML or JSON.

## Patentansprüche

1. Kommunikationssystem, welches aufweist:
➢ eine mobiles-Endgerät (1)-Anwendung, die eine einem Patienten (10) gewidmete elektronische Gesundheitsaufzeichnung in einem Standardformat verwaltet,
➢ ein erstes medizinisches Informationszentrum (2), das erste medizinische Daten in Bezug auf den Patienten (10) in einem ersten spezifischen Format speichert,
➢ ein zweites medizinisches Informationszentrum (3), das zweite medizinische Daten in Bezug auf den Patienten (10) in einem zweiten spezifischen Format speichert,
wobei es auch aufweist:
➢ einen ersten Zugang (21) zwischen dem ersten medizinischen Informationszentrum (2) und der mobiles-Endgerät (1)-Anwendung, der einen ersten Formatumwandler von dem ersten spezifischen Format in das Standardformat enthält,
➢ einen zweiten Zugang (31) zwischen dem zweiten medizinischen Informationszentrum (3) und der mobiles-Endgerät (1)-Anwendung, der einen zweiten Formatumwandler von dem zweiten spezifischen Format in das Standardformat enthält,
wobei es derart implementiert ist, dass:
➢ die mobiles-Endgerät (1)-Anwendung im Standardformat die ersten medizinischen Daten von dem ersten medizinischen Informationszentrum (2) über eine durch den ersten Zugang (21) gesendete erste Anfrage, und die zweiten medizinischen Daten von dem zweiten medizinischen Informationszentrum (3) über eine durch den zweiten Zugang (31) gesendete zweite Anfrage herunterladen kann, und die ersten medizinischen Daten und die zweiten medizinischen Daten in der elektronischen Gesundheitsaufzeichnung speichern kann, um die elektronische Gesundheitsaufzeichnung zu aktualisieren,
➢ der Patient (10) die aktualisierte elektronische Gesundheitsaufzeichnung über die mobiles-Endgerät (1)-Anwendung konsultieren kann,
➢ der Patient (10) von sich aus, über die mobiles-Endgerät (1)-Anwendung, die ersten und zweiten medizinischen Daten nicht modifizieren oder löschen kann, sobald sie in der mobiles-Endgerät (1)-Anwendung gespeichert sind, um die aktualisierte elektronische Gesundheitsaufzeichnung sicher zu machen,
wobei das System **dadurch gekennzeichnet, dass**:
➢ der erste Formatumwandler auch von dem Standardformat in das erste spezifische Format umwandelt,
➢ der zweite Formatumwandler auch von dem Standardformat in das zweite spezifische Format umwandelt,
und wobei es derart implementiert ist, dass:
➢ das erste medizinische Informationszentrum (2) in einem Normalmodus von der aktualisierten elektronischen Gesundheitsaufzeichnung der mobiles-Endgerät (1)-Anwendung im Standardformat ein gemeinsames Teil der zweiten medizinischen Daten über eine durch den ersten Zugang (21) gesendete dritte Anfrage herunterladen kann, und im ersten spezifischen Format den gemeinsamen Teil der zweiten medizinischen Daten in dem ersten medizinischen Informationszentrum (2) speichern kann, um das erste medizinische Informationszentrum (2) zu aktualisieren,
➢ das erste medizinische Informationszentrum (2) in einem Normalmodus von der aktualisierten elektronischen Gesundheitsaufzeichnung der mobiles-Endgerät (1)-Anwendung ein geheimes Teil der zweiten medizinischen Daten in einem beliebigen Format nicht herunterladen kann,
➢ das zweite medizinische Informationszentrum (3) in einem Normalmodus von der aktualisierten elektronischen Gesundheitsaufzeichnung der mobiles-Endgerät (1)-Anwendung im Standardformat ein gemeinsames Teil der ersten medizinischen Daten über eine durch den zweiten Zugang (31) gesendete vierte Anfrage herunterladen kann, und im zweiten spezifischen Format das gemeinsame Teil der ersten medizinischen Daten in dem zweiten medizinischen Informationszentrum (3) speichern kann, um das zweite medizinische Informationszentrum (3) zu aktualisieren,
➢ das zweite medizinische Informationszentrum (3) in einem Normalmodus von der aktualisierten elektronischen Gesundheitsaufzeichnung der mobiles-Endgerät (1)-Anwendung ein geheimes Teil der ersten medizinischen Daten in einem beliebigen Format nicht herunterladen kann,
➢ der Patient (10) sowohl die gemeinsamen als auch geheimen Teile beider erster und zweiter medizinischer Daten in der aktualisierten elektronischen Gesundheitsaufzeichnung über die mobiles-Endgerät (1)-Anwendung konsultieren kann,
und wobei es derart implementiert ist, dass:
➢ das erste medizinische Informationszentrum (2) in einem dringenden Modus von der aktualisierten elektronischen Gesundheitsaufzeichnung der mobiles-Endgerät (1)-Anwendung im Standardformat sowohl die gemeinsamen als auch die geheimen Teile der zweiten medizinischen Daten über eine durch den ersten Zugang (21) gesendete fünfte Anfrage herunterladen kann, um mit Dringlichkeit mit dem Patienten (10) umzugehen,
➢ das zweite medizinische Informationszentrum (3) in einem dringenden Modus von der aktualisierten elektronischen Gesundheitsaufzeichnung der mobiles-Endgerät (1)-Anwendung im Standardformat sowohl die gemeinsamen als auch die geheimen Teile der ersten medizinischen Daten über eine durch den zweiten Zugang (31) gesendete sechste Anfrage herunterladen kann, um mit Dringlichkeit mit dem Patienten (10) umzugehen.

2. Kommunikationssystem nach Anspruch 1, wobei:
➢ die medizinischen Informationszentren (2, 3) von einem oder mehreren Krankenhäusern verwaltet werden.

3. Kommunikationssystem nach Anspruch 2, wobei:
➢ die elektronische Gesundheitsaufzeichnung in einem der medizinischen Informationszentren (2, 3) nicht vollständig gespeichert wird, bevorzugt nicht vollständig in irgendeinem anderen von einem Krankenhaus verwalteten medizinischen Informationszentrum.

4. Kommunikationssystem nach einem der vorhergehenden Ansprüche, wobei es derart implementiert ist, dass:
➢ die mobile Endgerät (1)-Anwendung elektronische Gesundheitsaufzeichnungen, die jeweils unterschiedlichen Patienten gewidmet sind, im Standardformat verwaltet,
➢ jeder Patient (10) nur seine eigene aktualisierte elektronische Gesundheitsaufzeichnung über die mobiles-Endgerät (1)-Anwendung konsultieren kann, aber ein beliebiges Teil beliebiger medizinischer Daten aktualisierter elektronischer Gesundheitsaufzeichnungen eines anderen Patienten über die mobiles-Endgerät (1)-Anwendung nicht konsultieren kann.

5. Kommunikationssystem nach einem der vorhergehenden Ansprüche, wobei jedes der medizinischen Informationszentren (2, 3) aufweist:
➢ eine Speichervorrichtung (20, 23, 30, 33), die die medizinischen Daten in dem spezifischen Format speichert,
➢ einen Verbinder (24, 25, 34, 35), der den Formatumwandler enthält, ausgelegt, um die medizinischen Daten von der Speichervorrichtung (20, 23, 30, 33) in dem spezifischen Format zu erlangen und sie in das Standardformat umzuwandeln, bevor sie gesendet werden,
➢ einen Desktop (6, 7), der dazu ausgelegt ist, um von dem Verbinder (24, 25, 34, 35) eine umgewandelte medizinische Datensendung eines Patienten anzufordern und ferner diese, bevorzugt drahtlos, zu dem mobilen Endgerät (1) des Patienten zu senden.

6. Kommunikationssystem nach Anspruch 5, wobei:
➢ der Verbinder (24, 25, 34, 35) den Formatumwandler enthält, der dazu ausgelegt ist, die medizinischen Daten von dem Desktop (6, 7) in dem Standardformat zu empfangen und sie in das spezifische Format umzuwandeln, bevor sie zu der Speichervorrichtung (20, 30) gesendet werden,
➢ der Desktop (6, 7) dazu ausgelegt ist, um zu dem Verbinder (24, 25, 34, 35) medizinische Daten des Patienten zu senden, nach er sie von dem mobilen Endgerät (1) des Patienten, bevorzugt drahtlos, empfangen hat.

7. Kommunikationssystem nach Anspruch 5 oder 6, wobei:
➢ die drahtlose Verbindung Bluetooth oder Nahfeldkommunikation ist.

8. Kommunikationssystem nach einem der Ansprüche 5 bis 7, wobei:
➢ die drahtlose Kommunikation direkt ist, ohne durch irgendein öffentliches Netzwerk hindurchzugehen, bevorzugt ohne durch ein beliebiges Netzwerk hindurchzugehen.

9. Kommunikationssystem nach einem der vorhergehenden Ansprüche, wobei:
➢ zumindest die ersten und zweiten Anfragen, bevorzugt zumindest die ersten und zweiten und dritten und vierten Anfragen, besonders bevorzugt alle Anfragen, ausgeführt werden, indem vorbestimmte Masken ausgefüllt und gesendet werden.

10. Kommunikationssystem nach einem der vorhergehenden Ansprüche, wobei:
➢ zumindest eines des spezifischen Formats SOAP oder REST oder FILE ist,
➢ bevorzugt die spezifischen Formate aus einer Liste ausgewählt werden, die aus "SOAP, REST, FILE" besteht.

11. Kommunikationssystem nach einem der vorhergehenden Ansprüche, wobei:
➢ das Standardformat XML oder JSON ist.

## Revendications

1. Système de communication comprenant :
➢ une application de terminal mobile (1) gérant un dossier patient informatisé dédié à un patient (10), dans un format standard,
➢ un premier centre d'informations médicales (2), stockant des premières données médicales associées audit patient (10), dans un premier format spécifique,
➢ un deuxième centre d'informations médicales (3), stockant des deuxièmes données médicales associées audit patient (10), dans un deuxième format spécifique,
dans lequel il comprend également :
➢ une première passerelle (21), entre ledit premier centre d'informations médicales (2) et ladite application de terminal mobile (1), comportant un premier convertisseur de format dudit premier format spécifique audit format standard,
➢ une deuxième passerelle (31), entre ledit deuxième centre d'informations médicales (3) et ladite application de terminal mobile (1), comportant un deuxième convertisseur de format dudit deuxième format spécifique audit format standard,
dans lequel il est mis en oeuvre de sorte que :
➢ ladite application de terminal mobile (1) puisse télécharger, dans le format standard, lesdites premières données médicales à partir dudit premier centre d'informations médicales (2) via une première requête envoyée par le biais de ladite première passerelle (21) et lesdites deuxièmes données médicales à partir dudit deuxième centre d'informations médicales (3) via une deuxième requête envoyée par le biais de ladite deuxième passerelle (31), et puisse stocker lesdites premières données médicales et lesdites deuxièmes données médicales dans ledit dossier patient informatisé, de façon à mettre à jour ledit dossier patient informatisé,
➢ ledit patient (10) puisse consulter ledit dossier patient informatisé mis à jour via ladite application de terminal mobile (1),
➢ ledit patient (10) ne puisse pas lui-même, via ladite application de terminal mobile (1), modifier ou annuler lesdites premières et deuxièmes données médicales, une fois celles-ci stockées dans ladite application de terminal mobile (1), de façon à sécuriser ledit dossier patient informatisé mis à jour,
le système étant **caractérisé en ce que** :
➢ ledit premier convertisseur de format convertit également dudit format standard audit premier format spécifique,
➢ ledit deuxième convertisseur de format convertit également dudit format standard audit deuxième format spécifique,
et dans lequel il est mis en oeuvre de sorte que :
➢ ledit premier centre d'informations médicales (2) puisse télécharger, dans un mode ordinaire, à partir du dossier patient informatisé mis à jour de ladite application de terminal mobile (1), dans le format standard, une partie partagée desdites deuxièmes données médicales via une troisième requête envoyée par le biais de ladite première passerelle (21), et puisse stocker, dans un premier format spécifique, ladite partie partagée des deuxièmes données médicales, dans ledit premier centre d'informations médicales (2), de façon à mettre à jour ledit premier centre d'informations médicales (2),
➢ ledit premier centre d'informations médicales (2) ne puisse pas télécharger, dans un mode ordinaire, à partir du dossier patient informatisé mis à jour de ladite application de terminal mobile (1), dans un format quelconque, une partie confidentielle desdites deuxièmes données médicales,
➢ ledit deuxième centre d'informations médicales (3) puisse télécharger, dans un mode ordinaire, à partir du dossier patient informatisé mis à jour de ladite application de terminal mobile (1), dans le format standard, une partie partagée desdites premières données médicales via une quatrième requête envoyée par le biais de ladite deuxième passerelle (31), et puisse stocker, dans un deuxième format spécifique, ladite partie partagée des premières données médicales, dans ledit deuxième centre d'informations médicales (3), de façon à mettre à jour ledit deuxième centre d'informations médicales (3),
➢ ledit deuxième centre d'informations médicales (3) ne puisse pas télécharger, dans un mode ordinaire, à partir du dossier patient informatisé mis à jour de ladite application de terminal mobile (1), dans un format quelconque, une partie confidentielle desdites premières données médicales,
➢ ledit patient (10) puisse consulter à la fois les parties partagées et confidentielles des premières et deuxièmes données médicales dans ledit dossier patient informatisé mis à jour de ladite application de terminal mobile (1),
et dans lequel il est mis en oeuvre de sorte que :
➢ ledit premier centre d'informations médicales (2) puisse télécharger, dans un mode urgence, à partir du dossier patient informatisé mis à jour de ladite application de terminal mobile (1), dans le format standard, lesdites parties partagée et confidentielle desdites deuxièmes données médicales via une cinquième requête envoyée par le biais de ladite première passerelle (21), de façon à pouvoir traiter une urgence concernant ledit patient (10),
➢ ledit deuxième centre d'informations médicales (3) puisse télécharger, dans un mode urgence, à partir du dossier patient informatisé mis à jour de ladite application de terminal mobile (1), dans le format standard, lesdites parties partagée et confidentielle desdites premières données médicales via une sixième requête envoyée par le biais de ladite deuxième passerelle (31), de façon à pouvoir traiter une urgence concernant ledit patient (10).

2. Système de communication selon la revendication 1, dans lequel :
➢ lesdits centres d'informations médicales (2, 3) sont gérés par un ou plusieurs hôpitaux.

3. Système de communication selon la revendication 2, dans lequel :
➢ ledit dossier patient informatisé n'est stocké dans son intégralité dans aucun desdits centres d'informations médicales (2, 3), de préférence n'est stocké dans son intégralité dans aucun autre centre d'informations médicales géré par un hôpital.

4. Système de communication selon l'une quelconque des revendications précédentes, dans lequel il est mis en oeuvre de sorte que :
➢ ladite application de terminal mobile (1) gère des dossiers patient informatisés dédiés respectivement à des patients différents, dans le format standard,
➢ chaque patient (10) puisse consulter uniquement son propre dossier patient informatisé mis à jour via ladite application de terminal mobile (1), mais ne puisse consulter aucune partie de données médicales du dossier patient informatisé mis à jour d'un autre patient via ladite application de terminal mobile (1).

5. Système de communication selon l'une quelconque des revendications précédentes, dans lequel chacun des deux centres d'informations médicales (2, 3) comprend :
➢ un dispositif de stockage (20, 23, 30, 33) stockant lesdites données médicales, dans ledit format spécifique,
➢ un connecteur (24, 25, 34, 35) comportant ledit convertisseur de format, adapté pour obtenir lesdites données médicales à partir dudit dispositif de stockage (20, 23, 30, 33), dans ledit format standard, et pour les convertir dans ledit format standard, avant de les transmettre,
➢ un bureau (6, 7) adapté pour demander audit connecteur (24, 25, 34, 35) la transmission des données médicales du patient converties et pour les transmettre ensuite audit terminal mobile (1) dudit patient de préférence sans fil.

6. Système de communication selon la revendication 5, dans lequel :
➢ ledit connecteur (24, 25, 34, 35) comporte ledit convertisseur de format qui est adapté pour recevoir lesdites données médicales en provenance dudit bureau (6, 7), dans ledit format standard, et pour les convertir dans ledit format spécifique, avant de les transmettre audit dispositif de stockage (20, 30),
➢ ledit bureau (6, 7) est adapté pour transmettre audit connecteur (24, 25, 34, 35) les données médicales du patient après les avoir reçues en provenance du terminal mobile (1) dudit patient de préférence sans fil.

7. Système de communication selon la revendication 5 ou 6, dans lequel :
➢ ladite communication sans fil est une communication Bluetooth ou en champ proche.

8. Système de communication selon l'une quelconque des revendications 5 à 7, dans lequel :
➢ ladite communication sans fil est directe sans passer par un réseau public, de préférence sans passer par un quelconque réseau.

9. Système de communication selon l'une quelconque des revendications précédentes, dans lequel :
➢ au moins lesdites première et deuxième requêtes, de préférence au moins lesdites première et deuxième et troisième et quatrième requêtes, de manière davantage préférée toutes les requêtes, sont réalisées en remplissant et en envoyant des modèles prédéterminés.

10. Système de communication selon l'une quelconque des revendications précédentes, dans lequel :
➢ au moins un parmi lesdits formats spécifiques est SOAP, REST ou FILE,
➢ de préférence lesdits formats spécifiques sont choisis dans une liste constituée de « SOAP, REST, FILE ».

11. Système de communication selon l'une quelconque des revendications précédentes, dans lequel :
> ledit format standard est XML ou JSON.
